# EUROPEAN PATENT APPLICATION

(11) **EP 4 786 609 A2**
(43) Date of publication of application: **05.08.2026**
(21) Application number: 26154701.2
(22) Date of filing: 28.01.2026
(51) Int. Cl.: C12Q 1/6883

(54) **METHOD FOR DIAGNOSING MITRAL VALVE MYXOMATOUS DISEASE**

(30) Priority: 03.02.2025 IT 202500001866
(71) Applicant: UNIVERSITA' DEGLI STUDI DI MILANO, 20122 Milano (IT)
(72) Inventor: Lecchi, Cristina, 20122 Milano (IT); Brambilla, Paola Giuseppina, 20122 Milano (IT); Polli, Michele, 20122 Milano (IT); Bagardi, Mara, 20122 Milano (IT); Ghilardi, Sara, 20122 Milano (IT)
(74) Representative: Currado, Luisa

(57) **Abstract**

Disclosed is a miRNA panel that can be used as a prognostic marker and related methods and kits for diagnosing the risk of suffering from, the presence or absence and degree of development or severity of myxomatous mitral valve disease in dogs, by evaluating the expression levels of specific miRNAs.

## Description

### FIELD OF THE INVENTION

The present invention relates to the field of veterinary medicine and diagnostic biotechnology in that it relates to a novel microRNA (miRNA) panel, which can be used as a prognostic marker, and methods and kits for diagnosing the risk of suffering from, the presence or absence of, and the degree of development or severity of myxomatous mitral valve disease in dogs, by assessing the expression levels of specific miRNAs.

### TECHNICAL BACKGROUND

ACVIM (American College of Veterinary Internal Medicine) has proposed a classification of dogs suffering from myxomatous mitral valve disease (or myxomatous mitral valve degeneration MMVD or chronic valve degeneration) based on clinical parameters and diagnostic findings (Keene BW et al. 2019)

The dogs affected by MMVD are divided into 5 classes.

Class A identifies dogs at high risk of developing heart disease but who currently do not have an identifiable structural disorder of the heart (e.g., any Cavalier King Charles Spaniel or other predisposed breed without a heart murmur).

Class B identifies dogs with structural heart disease but who have never developed clinical signs caused by heart failure.

Class B1 describes asymptomatic dogs that exhibit echocardiographic signs of valvular degeneration in the absence of radiographic and echocardiographic signs of left atrioventricular remodeling or that echocardiographically exhibit only left atrial or ventricular remodeling.

Class B2 describes asymptomatic dogs exhibiting echocardiographic signs of valvular degeneration associated with radiographic and echocardiographic signs of left atrioventricular remodeling.

Class C indicates dogs with current or past clinical signs of heart failure caused by MMVD.

Class D refers to dogs with end-stage MMVD, where clinical signs of cardiac failure are refractory to standard treatment (defined later in this consent statement).

The aforementioned classes can also be found as called ACVIM A B B1 B2 C and D where the acronym is the ACVIM (American College of Veterinary Internal Medicine) that classified them, which is therefore analogous to the name MMVD A B B1 B2 C and D.

The microRNAs are non-coding RNA molecules involved in the regulation of gene transcription. Their presence in the blood is associated with the clinical condition of the patient, so the types and levels of circulating microRNAs vary depending on the pathophysiological status of the patient.

The identification of dogs with early asymptomatic MMVD through evaluation of miR-30b-5p that is known in tumor progression has been described (Bagardi et al., 2022).

Yang et al. (2017), using a *targeted* approach, described that the expression of some miRNAs carried by microvesicles, in particular cfa-miR-9, cfa-miR-181c, cfa-miR-495 and cfa-miR-599, changes with the progression of MMVD and the development of heart failure, but also changes as a function of age.

Ghilardi et al. (2022) through a statistical analysis describes a pilot study that highlights the relationship between high levels of miR-30b-5p and forms of MMVD that appear echocardiographically more stable over time in Cavalier King Charles Spaniel dogs.

Ro et al. (2021), using a *targeted* approach, describes that up-regulation of cfa-miR-130b is observed in dogs with stage B MMVD while cfa-miR-375 and cfa-let-7b are up-regulated in dogs with cardiac hypertrophy.

Jung and Bohan (2017) describe the characterization of circulating microRNA expression profiles by whole genome sequencing for dogs with congestive heart failure (CHF) secondary to myxomatous mitral valve degeneration (MMVD) and genetic analysis revealed distinct expression patterns of circulating microRNAs between healthy dogs and dogs with CHF, in particular, upregulation of miR-133, miR-1, miR-let-7e and miR-125 and downregulation of 4 miR-30c, miR-128, miR-142 and miR-423. Two groups of dogs referable to the classification ACVIM A (healthy and predisposed) and ACVIM C (advanced and severe form) are included in the study.

Yang et al. (2018) describes variations in miRNA expression in an *in-vitro* model of valvular interstitial cells (VICs) and has highlighted that VICs from diseased mitral valves show significant down-regulation of let-7c, miR-17, miR-20a and miR-30d.

Li et al 2015 describes that dogs with MMVD class B1/B2 or C/D had four up-regulated miRNAs, of which three members of the family cfa-let-7/cfa-miR-98, while seven other miRNAs were downregulated compared to class A subjects, the expression of six of the 11 miRNAs was significantly different between dogs in phase C/D and those in phase B1/B2 and the changes in their expression were more marked with the progression of the severity of the disease.

The microRNA panel identified by the inventors of the present invention was identified by an "omical" approach of *untargeted* sequencing by NGS, Next Generation Sequencing, parallel sequencing, i.e. blood samples were taken from 3 groups of patients (ACVIM A, B1 and B2) and all miRNAs present in the samples were identified and quantified. Through a statistical analysis, the miRNAs differentially expressed between the 3 groups were then identified, identifying those specific to each group. The miRNA panel object of the present invention was then defined using an approach and a model different from those reported in the literature that used either a *targeted* approach, quantifying only some miRNAs previously reported in the literature for other heart diseases or other species, but which are not specific for dog MMVD (as in Bagardi et al., 2022; Yang et al., 2017; Ghilardi et al., 2022; Ro et al., 2021; Li et al., 2015); or an untageted approach *(not targeted),* but including ACVIM A vs C groups, i.e. patients with severe and advanced mitral valve degeneration, to diagnose which it is not necessary to resort to a molecular marker (as described in Jung and Bohan, 2017). Echocardiographic examination allows the diagnosis and follow-up of patients with advanced degeneration (B2, C, and D).

Thus, since first-stage degeneration (ACVIM B1) is hardly detectable and few veterinarians are specialized in cardiology, that is, they have the skills and experience to detect the pathology in the early stages of degeneration development, the microRNA panel of the present invention is particularly useful.

Although fragmentary data relating to miRNAs in in canine MMVD are present in literature, they are different, fragmentary and not overlapping with what is the subject of the present patent application, both in terms of selected miRNAs, methodological approach, and claimed technical function.

For example Li et al. (2015) identified a panel of differentially expressed miRNAs by comparing the following clinical groups: B1/B2 versus A, C/D versus A, and C/D versus B1/B2 being different from those of the present application. Moreover, Li et al. (2015) do not carry out any analysis aimed at evaluating miRNAs as diagnostic, prognostic or clinical stratification biomarkers, limiting themselves to discussing a possible biological role as molecular modulators, and the study of Li et al. (2015) does not distinguish between ACVIM stages B1 and B2, considering stage B as a single group, without addressing the clinically relevant issue of discrimination between early preclinical and advanced preclinical stage.

Bagardi et al. (2022) identified only one miRNA (miR-30b-5p) as differentially expressed miRNAs in in canine MMVD, and it is not proposed as part of a biomarker panel.

Palarea-Albaladejo et al. (2024) tested several miRNAs associated with the various ACVIM stages. only one (miR-206-3p) overlaps with the present application.

Yang et al. (2018) investigated MMVD-associated miRNAs using an in vitro model based on valvular interstitial cells (VICs). This experimental approach is conceptually and technically distinct from that of the present patent application, which is based on biological fluids. MiRNAs identified in a cell model are not directly transferable or usable as in vivo biomarkers for patient screening, clinical clustering, or follow-up.

Yang et al. (2017) analyzed miRNAs carried by exosomes circulating in the blood of dogs with MMVD. Among the differentially expressed miRNAs, only one (miR-138) is in common with those of the present application. Furthermore, the authors do not classify patients according to the ACVIM guidelines, but generically divide them into "asymptomatic MMVD" and "MMVD with history of CHF", making the comparison with the clinical stratification proposed in the present application nonoverlapping.

The present proposes an integrated miRNA panel with a clear technical application as biomarkers; allows, in an innovative and non-obvious way, the discrimination between the ACVIM B1 and B2 stadiums, an aspect never reported before.

Therefore, although the present application uses miRNAs as biomarkers capable of discriminating ACVIM A, B, C and D stages, the proposed panel does not present significant overlaps with what is reported in the state of the art, neither from the point of view of the selected miRNAs nor from that of the claimed technical function.

### SUMMARY OF THE INVENTION

10% of the canine population suffers from heart disease and, of this 10%, 75% is affected by mitral valve disease. To date, the diagnosis of mitral valve disease in dogs is carried out through a clinical evaluation by a veterinarian and an echocardiogram. The gold standard for the diagnosis of mitral valve disease in dogs is the echocardiographic examination, a very widespread, well standardized and non-invasive method that allows obtaining essential information for the correct classification of the subject and that is useful for the clinician to decide the type of drug therapy to which to subject the individual animal (Thomas et al., 1993; Keene et al., 2019; Acierno et al., 2018; Rishniw, 2018). However, this examination is often proposed when clinical signs are already present at the clinical examination, such as a heart murmur identified on auscultation, allowing to highlight lesions already developed at the valve level, without providing information regarding the possible evolution of the disease over time.

Some dog breeds, such as the Cavalier King Charles Spaniel and the Chihuahua, and, in general, small-medium dogs (under 20 kg), have sometimes very rapid evolutions, with a risk of developing heart failure even at a young age, under 4 years (Borgarelli and Buchanan, 2012). Echocardiographic examination allows the diagnosis and follow-up of these patients, but since degeneration in the early stages is hardly detectable and few veterinarians are specialized in cardiology, that is, they have the skills and experience to detect the pathology in the early stages of development, the inventors of the present invention have identified a panel of circulating microRNAs specific to mitral valve pathology in dogs and this panel of biomarkers allows an early diagnosis and staging of the pathology by means of a simple blood sample to be carried out by liquid biopsy. Moreover, thanks to the fact that they are quantifiable in the blood, they can be evaluated in a minimally invasive and repeatable way over time, thus allowing the development of the disease to be followed.

The inventors of the present invention identified and selected a novel microRNA panel by a specific screening in dogs which proved to have specific distinguishing features:
- Isolated blood samples are used, so the test is minimally invasive because it is a liquid biopsy, that is, quantification in biological fluids that can be repeated over time (follow up), which allows the quantitative evaluation of the evolution of the disease;
- the microRNA panel varies depending on the progress of the disease, thus allowing it to be staged in real time and quantitatively;
- the microRNA panel varies long before it is possible to make the diagnosis by echocardiographic examination, thus allowing an early diagnosis of the first stage (ACVIM B1), which is hardly recognizable by a veterinarian without specialization in cardiology;
- the microRNA panel allows early diagnosis (ACVIM B1) even in very young dogs under 3 years of age;
- the microRNAs are very stable and resistant to degradation, therefore it is not necessary to quantify them immediately after the collection of the matrix, but it is possible to comply with the timing of a diagnostic laboratory;
- molecular diagnosis through the microRNA panel allows to define more specific therapies for each patient, since it guarantees an accurate classification of patients suffering from the same pathology, also paving the way in veterinary medicine for personalized medicine.

The miRNA panel of the present invention may be employed as a prognostic marker and in a non-invasive diagnostic method thereof.

The miRNA panel is not derived from miRNAs known in other species, for example human and mouse, which have been translated and therefore used for diagnosis in dogs, but has been identified by characterizing the profile of miRNAs circulating in the plasma of dogs enrolled in a specific study. For this reason it is specific to the genus *canis.*

The fact that the miRNA panel, on which the present invention is based, has been specifically identified in dogs, and no known miRNAs have been employed in similar diseases and in different species, means that the method of the present invention is highly specific and predictive.

The proposed microRNA panel was identified by an OMICO approach of *untargeted* sequencing (NGS, Next Generation Sequencing), i.e. blood was collected from 3 groups of patients (ACVIM A, B1 and B2) and all miRNAs present in the samples were identified and quantified. Through a statistical analysis, the miRNAs differentially expressed between the 3 groups were then identified, identifying those specific to each group.

Further features of the present invention will be clear from the following detailed description with reference to the provided experimental examples.

### DETAILED DESCRIPTION OF THE INVENTION

In the context of the present invention myxomatous mitral valve disease is synonymous with myxomatous mitral valve degeneration (MMVD) or chronic valve degeneration.

In the scope of the present invention, MMVD class A or MMVD A means absence of degeneration of the mitral valve, therefore healthy subjects.

In the context of the present invention, MMVD class B or MMVD B means degeneration of the mitral valve characterized by structural heart disease but without clinical signs caused by heart failure.

In the context of the present invention, MMVD class B1 or MMVD B1 means degeneration of the mitral valve characterized by lack of radiographic and echocardiographic evidence of left atrioventricular remodeling or with only left atrial or ventricular remodeling that can be evidenced echocardiographically.

In the context of the present invention, MMVD class B2 or MMVD B2 means degeneration of the asymptomatic mitral valve that presents echocardiographic signs of valve degeneration associated with radiographic and echocardiographic signs of left atrioventricular remodeling.

Within the scope of the present invention, variation in expression levels means under- or over-expression with respect to a control that is preferably the healthy subject or MMVD group A.

An object of the present invention is a miRNA panel consisting of the following miRNAs:

| **SEQ. ID. NO.** | **NAME** | **SEQUENCE** | **ST 26 STANDARD** |
|---|---|---|---|
| **SEQ. ID NO. 1** | cfa-miR-138b-5p | AGCUGGUGUUGUGAAUCAUGCCGA | AGCTGGTGTTGTGAATCATGCCGA |
| **SEQ. ID NO.2** | cfa-miR-144-5p | GGAUAUCAUCAUAUACUGUAAG | GGATATCATCATATACTGTAAG |
| **SEQ. ID NO.3** | cfa-miR-150-5p | UCUCCCAACCCUUGUACCAGUG | TCTCCCAACCCTTGTACCAGTG |
| **SEQ. ID NO.4** | cfa-miR-182-3p | UGGUUCUAGACUUGCCAACUA | TGGTTCTAGACTTGCCAACTA |
| **SEQ. ID NO.5** | cfa-miR-206-3p | UGGAAUGUAAGGAAGUGUGUGGG | TGGAATGTAAGGAAGTGTGTGG |
| **SEQ. ID NO.6** | cfa-miR-207-3p | GCUUCUCCGGUCUCUCCUCCUUC | GCTTCTCCGGTCTCTCCTCCTTC |
| **SEQ. ID NO.7** | cfa-miR-33b-5p | GUGCAUUGCUGUUGCAUUGC | GTGCATTGCTGTTGCATTGC |
| **SEQ. ID NO.8** | cfa-miR-346-5p | UGUCUGCCCGCAUGCCUGCCUCU | TGTCTGCCCGCATGCCTGCCTCT |
| **SEQ. ID NO.9** | cfa-miR-365-1-5p | AGGGACUUUUGGGGGCAGAU | AGGGACTTTTGGGGGCAGAT |
| **SEQ. ID NO.10** | cfa-miR-493-5p | UGUACAUGGUAGGCUUUCAUU | TGTACATGGTAGGCTTTCATT |
| **SEQ. ID NO.11** | cfa-miR-632-5p | CUGACGGGAGCAGAGCGGCGAA | CTGACGGGAGCAGAGCGGCGAA |
| **SEQ. ID NO.12** | cfa-miR-8794-1-5p | AAUCGGCAAGUGGCCUGGGGAC | AATCGGCAAGTGGCCTGGGGAC |
| **SEQ. ID NO.13** | cfa-miR-8875-3p | AUUGUCUUUGGGUUCUUAGCCU | ATTGTCTTTGGGTTCTTAGCCT |
| **SEQ. ID NO.14** | cfa-miR-92b-3p | UAUUGCACUCGUCCCGGCCUCC | TATTGCACTCGTCCCGGCCTCC |
| **SEQ. ID NO.15** | cfa-miR-92b-5p | AGGGACGGGACGCGGUGCAGU | AGGGACGGGACGCGGTGCAGT |
| **SEQ. ID NO.16** | cfa-miR-95-3p | UUCAACGGGUAUUUAUUGAGCA | TTCAACGGGTATTTATTGAGCA |

Preferably the miRNA panel consists of the following miRNAs: cfa-miR-144-5p SEQ. ID NO.2, cfa-miR-206-3p SEQ. ID NO.5, cfa-miR-207-3p SEQ. ID NO.6, cfa-miR-365-1-5p SEQ. ID NO.9, cfa-miR-493-5p SEQ. ID NO.10, cfa-miR-632-5p SEQ. ID NO.11.

Preferably the miRNA panel consists of the following miRNAs: cfa-miR-207-3p SEQ. ID NO.6, cfa-miR-33b-5p SEQ. ID NO.7, and cfa-miR-632-5p SEQ. ID NO.11.

Preferably the miRNA panel consists of the following miRNAs: cfa-miR-144-5p SEQ. ID NO.2, cfa-miR-150-5p SEQ. ID NO.3, cfa-miR-182-3p SEQ. ID NO.4, cfa-miR-206-3p SEQ. ID NO.5, cfa-miR-365-1-5p SEQ. ID NO.9, cfa-miR-493-5p SEQ. ID NO.10, cfa-miR-632-5p SEQ. ID NO.11, cfa-miR-8794-1-5p SEQ. ID NO.12, cfa-miR-8875-3p SEQ. ID NO.13.

Preferably the miRNA panel consists of the following miRNAs: cfa-miR-138b-5p SEQ. ID NO.1, cfa-miR-150-5p SEQ. ID NO.3, cfa-miR-346-5p SEQ. ID NO.8, cfa-miR-8875-3p SEQ. ID NO.13, cfa-miR-92b-3p seq. ID NO.14, cfa-miR-92b-5p SEQ. ID NO.15, cfa-miR-95-3p SEQ. ID NO.16.

A further object of the present invention is the use of at least one miRNA, preferably at least three miRNAs or belonging to the panel consisting of the following miRNAs:

| **SEQ. ID. NO.** | **NAME** | **SEQUENCE (ST 26 STANDARD)** |
|---|---|---|
| **SEQ. ID NO. 1** | cfa-miR-138b-5p | AGCTGGTGTTGTGAATCATGCCGA |
| **SEQ. ID NO.2** | cfa-miR-144-5p | GGATATCATCATATACTGTAAG |
| **SEQ. ID NO.3** | cfa-miR-150-5p | TCTCCCAACCCTTGTACCAGTG |
| **SEQ. ID NO.4** | cfa-miR-182-3p | TGGTTCTAGACTTGCCAACTA |
| **SEQ. ID NO.5** | cfa-miR-206-3p | TGGAATGTAAGGAAGTGTGTGG |
| **SEQ. ID NO.6** | cfa-miR-207-3p | GCTTCTCCGGTCTCTCCTCCTTC |
| **SEQ. ID NO.7** | cfa-miR-33b-5p | GTGCATTGCTGTTGCATTGC |
| **SEQ. ID NO.8** | cfa-miR-346-5p | TGTCTGCCCGCATGCCTGCCTCT |
| **SEQ. ID NO.9** | cfa-miR-365-1-5p | AGGGACTTTTGGGGGCAGAT |
| **SEQ. ID NO.10** | cfa-miR-493-5p | TGTACATGGTAGGCTTTCATT |
| **SEQ. ID NO.11** | cfa-miR-632-5p | CTGACGGGAGCAGAGCGGCGAA |
| **SEQ. ID NO.12** | cfa-miR-8794-1-5p | AATCGGCAAGTGGCCTGGGGAC |
| **SEQ. ID NO.13** | cfa-miR-8875-3p | ATTGTCTTTGGGTTCTTAGCCT |
| **SEQ. ID NO.14** | cfa-miR-92b-3p | TATTGCACTCGTCCCGGCCTCC |
| **SEQ. ID NO.15** | cfa-miR-92b-5p | AGGGACGGGACGCGGTGCAGT |
| **SEQ. ID NO.16** | cfa-miR-95-3p | TTCAACGGGTATTTATTGAGCA |

as a diagnostic marker, preferably of myxomatous mitral valve disease, preferably in dogs.

Preferably, at least one miRNA, preferably at least three miRNAs, more preferably all miRNAs of the panel consisting of: SEQ.ID.NO.1 to SEQ.ID.NO. 16 for use as a diagnostic marker of absence of MMVD (healthy subject MMVD stage A, MMVD A).

Preferably at least one miRNA, preferably at least three miRNAs, more preferably all miRNAs selected from the group consisting of: cfa-miR-144-5p SEQ.ID.NO.2, cfa-miR-206-3p SEQ.ID.NO.5, cfa-miR-207-3p SEQ.ID.NO.6, cfa-miR-365-1-5p SEQ.ID.NO.9, cfa-miR-493-5p SEQ.ID.NO.10, cfa-miR-632-5p SEQ.ID.NO.11, is a diagnostic marker of MMVD at stage B1 MMVD B1.

Preferably at least one miRNA, preferably at least three miRNAs, more preferably all miRNAs of the panel selected from the group consisting of: cfa-miR-207-3p SEQ.ID.NO.6, cfa-miR-33b-5p SEQ.ID.NO.7, and cfa-miR-632-5p SEQ.ID.NO.11, is a diagnostic marker of stage B1 MMVD in subjects under 3 years of age.

Preferably at least one miRNA of the panel selected from the group consisting of: cfa-miR-144-5p SEQ.ID.NO.2, cfa-miR-150-5p SEQ.ID.NO.3, cfa-miR-182-3p SEQ.ID.NO.4, cfa-miR-206-3p SEQ.ID.NO.5, cfa-miR-365-1-5p SEQ.ID.NO.9, cfa-miR-493-5p SEQ.ID.NO.10, cfa-miR-632-5p SEQ.ID.NO.11, cfa-miR-8794-1-5p SEQ.ID.NO.12, cfa-miR-8875-3p SEQ.ID.NO.13 is diagnostic marker of MMVD at stage B2.

More preferably a panel comprising cfa-miR-138b-5p SEQ.ID.NO.1, cfa-miR-150-5p SEQ.ID.NO.3, cfa-miR-346-5p SEQ.ID.NO.8, cfa-miR-8875-3p SEQ.ID.NO.13, cfa-miR-92b-3p SEQ.ID.NO.14, cfa-miR-92b-5p SEQ.ID.NO.15, cfa-miR-95-3p SEQ.ID.NO.16, is diagnostic marker of MMVD at stage B1 compared to MMVD at stage B2, then discriminates stage B1 from B2.

A further object of the present invention is a method for diagnosing the risk of suffering from, the presence or absence and the degree of development or severity of myxomatous disease of the mitral valve which provides for the identification and/or measurement of the expression level of at least one miRNA selected from the group consisting of: SEQ.ID.NO.1 to SEQ.ID.NO.16.
in an isolated sample of biological fluid obtained from said subject
wherein the presence and/or modified level of expression of said at least one miRNA relative to a control, indicates an increased risk to the subject of suffering, the presence or absence, degree of development or severity of myxomatous mitral valve disease
wherein said subject is a dog.

Preferably the method provides for the identification and/or measurement of the expression level of at least three miRNAs selected from the group consisting of or in the group consisting of: from SEQ.ID.NO.1 to SEQ.ID.NO. 16.

Preferably the biological fluid is selected from the group consisting of: blood, serum, plasma, urine, stool, saliva.

More preferably the biological fluid is blood, plasma, serum.

Blood, serum or plasma are the preferred biological samples as they flow into the heart and are in direct contact with the organ involved in the disease to be investigated.

Preferably the expression level is measured by amplifying the target miRNAs.

Preferably the control is an isolated sample obtained from a healthy subject or MMVD A or MMVD B1.

The expression level of a miRNA is evaluated with respect to the control, therefore variations in the expression levels of one or more miRNAs belonging to a miRNA panel is identifying the risk of suffering, the presence or absence and the degree of development or severity of myxomatous mitral valve disease.

Preferably the dog is of any breed or mongrel.

Preferably it is a dog less than 3 years of age.

Preferably it is a defined small dog i.e. a subject weighing less than 20 kg.

Most preferably the dog is a Cavalier King Charles Spaniel, Chihuahua, Dachshund, Shih Tzu, Border Collie, Poodle, Maltese, Yorkshire Terrier, Whippet, Miniature Schnauzer.

Preferably, the method to diagnose the risk of suffering, the presence or absence and the degree of development or severity of myxomatous mitral valve disease includes the following stages:
a) collection of an isolated sample of biological fluid from a subject at risk or with MMVD
b) extract the miRNAs from the sample obtained in stage a)
c) amplification of the individual miRNAs extracted in stage b), preferably by quantitative PCR
d) measurement of the level of expression of at least one miRNA selected from the group consisting of: from SEQ.ID.NO.1 to SEQ.ID.NO. 16
e) compare the presence and/or the modified expression level of said at least one miRNA referred to in step d) with respect to a control
wherein the presence and/or modified level of expression of at least one miRNA of step d) relative to the control indicates an increased risk to the subject of suffering from, the presence or absence, degree of development or severity of myxomatous disease of the mitral valve.

Preferably the control is an isolated sample obtained from a healthy subject or MMVD A or MMVD B1.

Preferably the variation in the expression level of at least one miRNA selected from the group of: SEQ.ID.NO.1 to SEQ.ID.NO. 16, compared to the control is indicative of the presence of the disease.

Preferably the variation in the expression level of at least one miRNA selected from the group consisting of: cfa-miR-144-5p SEQ.ID.NO.2, cfa-miR-206-3p SEQ.ID.NO.5, cfa-miR-207-3p SEQ.ID.NO.6, cfa-miR-365-1-5p SEQ.ID.NO.9, cfa-miR-493-5p SEQ.ID.NO.10, cfa-miR-632-5p SEQ.ID.NO.11, is indicative of MMVD at the ACVIM B1 stage.

More preferably the variation in the expression level of all miRNAs: cfa-miR-144-5p SEQ.ID.NO.2, cfa-miR-206-3p SEQ.ID.NO.5, cfa-miR-207-3p SEQ.ID.NO.6, cfa-miR-365-1-5p SEQ.ID.NO.9, cfa-miR-493-5p SEQ.ID.NO.10, cfa-miR-632-5p SEQ.ID.NO.11 is indicative of MMVD at the ACVIM B1 stage.

Preferably the variation in the expression level of at least one miRNA selected from the group consisting of: cfa-miR-207-3p SEQ.ID.NO.6, cfa-miR-33b-5p SEQ.ID.NO.7, and cfa-miR-632-5p SEQ.ID.NO.11, is indicative of MMVD at the ACVIM B1 stage in subjects under 3 years of age.

More preferably the variation in the expression level of all miRNAs cfa-miR-207-3p SEQ.ID.NO.6, cfa-miR-33b-5p SEQ.ID.NO.7, and cfa-miR-632-5p SEQ.ID.NO.11 is indicative of MMVD at the ACVIM B1 stage in subjects under 3 years of age.

Subjects under 3 years of age with ACVIM B1 stage MMVD have asymptomatic early degeneration without echocardiographic evidence of left atrioventricular remodeling and with minimal valve alterations.

Preferably the variation in the expression level of at least one miRNA selected from the group consisting of: cfa-miR-144-5p SEQ.ID.NO.2, cfa-miR-150-5p SEQ.ID.NO.3, cfa-miR-182-3p SEQ.ID.NO.4, cfa-miR-206-3p SEQ.ID.NO.5, cfa-miR-365-1-5p SEQ.ID.NO.9, cfa-miR-493-5p SEQ.ID.NO.10, cfa-miR-632-5p SEQ.ID.NO.11, cfa-miR-8794-1-5p SEQ.ID.NO.12, cfa-miR-8875-3p SEQ.ID.NO.13 is indicative of MMVD at the ACVIM B2 stage.

More preferably the variation in the expression level of cfa-miR-144-5p SEQ.ID.NO.2, cfa-miR-150-5p SEQ.ID.NO.3, cfa-miR-182-3p SEQ.ID.NO.4, cfa-miR-206-3p SEQ.ID.NO.5, cfa-miR-365-1-5p SEQ.ID.NO.9, cfa-miR-493-5p SEQ.ID.NO.10, cfa-miR-632-5p SEQ.ID.NO.11, cfa-miR-8794-1-5p SEQ.ID.NO.12, cfa-miR-8875-3p SEQ.ID.NO.13 is indicative of MMVD at the ACVIM B2 stage.

Preferably the variation in the expression level of all miRNAs cfa-miR-138b-5p, cfa-miR-150-5p, cfa-miR-346-5p, cfa-miR-8875-3p, cfa-miR-92b-3p, cfa-miR-92b-5p, cfa-miR-95-3p, allows to discriminate MMVD subjects at ACVIM B1 stage from those at ACVIM B2 stage.

More preferably, within the characteristic panel of ACVIM B1 pathology (cfa-miR-144-5p SEQ.ID.NO.2, cfa-miR-206-3p SEQ.ID.NO.5, cfa-miR-207-3p SEQ.ID.NO.6, cfa-miR-365-1-5p SEQ.ID.NO.9, cfa-miR-493-5p SEQ.ID.NO.10, cfa-miR-632-5p SEQ.ID.NO.11) is overexpressed compared to the MMVD group at the ACVIM A stage cfa-miR-493-5p SEQ.ID NO. 10, while they are under-expressed with respect to the ACVIM A group: cfa-miR-144-5p SEQ.ID NO. 2, cfa-miR-206-3p SEQ.ID NO.5, cfa-miR-207-3p SEQ.ID NO.6, cfa-miR-365-1-5p and cfa-miR-632-5p SEQ.ID NO.11.

More preferably, within the characteristic panel of MMVD at stage ACVIM B1 in subjects under 3 years of age: cfa-miR-207-3p SEQ.ID.NO.6, cfa-miR-33b-5p SEQ.ID.NO.7, and cfa-miR-632-5p SEQ.ID.NO.11 all three are under-expressed compared to ACVIM A.

More preferably, within the characteristic panel of MMVD at the ACVIM B2 stage (cfa-miR-144-5p, cfa-miR-150-5p, cfa-miR-182, cfa-miR-206-3p, cfa-miR-365-1-5p, cfa-miR-493-5p, cfa-miR-632-5p, cfa-miR-8794-1-5p, cfa-miR-8875-3p) are overexpressed compared to ACVIM A: cfa-miR-182-3p SEQ.ID NO.4, cfa-miR-493-5p SEQ.ID NO.10, cfa-miR-8794-1-5p SEQ.ID NO.12, cfa-miR-8875-3p SEQ.ID NO.13,
are under-expressed with respect to ACVIM A: cfa-miR-144-5p SEQ.ID NO.2), cfa-miR-150-5p SEQ.ID NO.3, cfa-miR-206-3p SEQ.ID NO.5, cfa-miR-365-1-5p SEQ.ID NO.9, cfa-miR-632-5p SEQ.ID NO.11.

More preferably, within the characteristic panel of MMVD at the ACVIM B2 stage (cfa-miR-144-5p, cfa-miR-150-5p, cfa-miR-182, cfa-miR-206-3p, cfa-miR-365-1-5p, cfa-miR-493-5p, cfa-miR-632-5p, cfa-miR-8794-1-5p, cfa-miR-8875-3p), cfa-miR-150-5p SEQ.ID NO.3 is under-expressed with respect to the ACVIM B1 stage, while cfa-miR-138b-5p SEQ.ID NO.1, cfa-miR-346-5p SEQ.ID NO.8, cfa-miR-8875-3p SEQ.ID No. 13, cfa-miR-92b-3p SEQ.ID NO. 14, cfa-miR-92b-5p SEQ.ID NO. 15, cfa-miR-95-3p SEQ.ID NO. 16 are overexpressed with respect to the ACVIM B1 stage.

MMVD subjects at ACVIM B1 stage are asymptomatic patients with echocardiographically evidenced valve degeneration but who do not present echocardiographic and radiographic evidence of left atrioventricular remodeling or who present only atrial or left ventricular remodeling, while ACVIM B2 subjects are always asymptomatic and present with valve degeneration associated with both atrial and left ventricular remodeling.

It is a further object of the present invention a kit for the in vitro evaluation of the risk of suffering, the presence or absence and the degree of development or severity of myxomatous disease of the mitral valve comprising swabs and reagents for extracting, amplifying and measuring the expression level of at least one miRNA selected from the group consisting of: SEQ.ID NO.1 TO SEQ.ID NO.16 and comparing it with a control which preferably represents a healthy subject.

Preferably, under-expression means expression of a value lower than a threshold value, which is the control, preferably healthy subject and/or ACVIM A subject.

Preferably, overexpression means expression of a higher value than the control, which is preferably the healthy subject and/or ACVIM A subject.

### EXAMPLES

### Materials and methods

### Sampling: clinical evaluation and echocardiogram.

Cavalier King dogs owned and visited at the Cardiology Unit of the Department of Veterinary Medicine and Animal Science (DIVAS) of the University of Milan were enrolled in the study. In accordance with the statement of the ethical committee of the University of Milan, the informed consent was signed by the owners, number 2/2016, and a high standard of care was provided during each visit.

Cardiological evaluation was performed on dogs fasted for at least 12 hours during a routine veterinary visit. The clinical data of the animals included the medical history and clinical and echocardiographic examinations. The cardiovascular system was assessed through verification of the presence/absence of murmurs by two operators with different levels of competence, respectively a PhD student in cardiology in the third year and a professor with more than twentyyears of practice in veterinary clinical cardiology. Results of auscultation included presence/absence, time and intensity of murmur (0 = absent; 1 = left or soft apical systolic I-II/VI; 2 = bilateral or moderate and strong systolic III-IV/VI; 3 = bilateral or palpable systolic V-VI/VI) (Rishniw, 2018). Blood pressure was measured indirectly by a Doppler method according to the ACVIM consensus statement (Acierno et al., 2018). Peripheral venous blood sampling was performed at the end of the examination. Blood was collected from the jugular or cephalic vein in two 2.5 ml EDTA tubes. A portion of the blood samples were used to perform routine blood tests to exclude any comorbidities. Echocardiographic examination was used to diagnose MMVD mitral valve disease. The standard transthoracic echocardiographic examination was performed with the My Lab50 Gold Cardiovascular ultrasound scanner (Esaote, Genoa, Italy), equipped with multifrequency phased array probes (3.5-5 and 7.5-10 MHz), chosen according to the weight of the subject. The video clips were acquired and stored with the ultrasound software for offline measurements (Thomas et al., 1993). All tests were performed without pharmacological constraint. Dogs were classified according to the ACVIM classification scheme (Acierno et al., 2018).

The inclusion criteria for dogs in the clinically normal group (ACVIM A) were: no echocardiographic evidence of heart disease, no clinical sign, no abnormality in the results of the complete blood count and biochemical analysis, and no history of medical treatment in the previous 6 months. The inclusion criteria for dogs with stage B1 MMVD were: no abnormality in the complete blood count and biochemical analysis results, echocardiographic evidence of thickened or prolapsed mitral valve and mitral valve regurgitation, no evidence of left atrial dilatation, defined as a left atrium to aortic root ratio (LA/Ao) <1.6 on two-dimensional echocardiography, and/or no left ventricular dilatation, defined as left ventricular diastolic diameter normalized for body weight (LVIDdN) <1.7. Dogs that presented with left atrial or ventricular remodeling were also included because they did not match the criteria of the current guidelines for ACVIM class B2 (Keene et al., 2019). The degree of mitral regurgitation was assessed using Doppler color by calculating the maximum signal ratio of the regurgitant jet area to the left atrial area (Chetboul & Tissier, 2012). The size of the regurgitant jet was estimated as the percentage of the left atrial area (at the nearest 5%) occupied by the largest jet. It was considered trivial or trace (<10%), mild (between 10 and 30%), moderate (between 30 and 70%), or severe (>70%) (Chetboul & Tissier, 2012; Ponikowski et al., 2016). More specifically, mitral regurgitation was considered trivial when the regurgitant jet was not detectable in all systolic events, while it was considered trace when it was always visible (Ponikowski et al., 2016). In addition, ACVIM class B1 dogs have not had a medical treatment history in the previous 6 months. Three groups of client-owned dogs were included in this study: group A (12 subjects), or healthy control, group B1 (24 subjects); group B2 (12 subjects).

### Extraction and sequencing of smallRNAs

Small RNAs were extracted using the Micro RNA Concentrator kit (A&A Biotechnology, cat. no. 035-25C) following the manufacturer's instructions. RNA quality and quantity were verified according to MIQE guidelines (Bustin et al., 2009). RNA concentration was quantified by Qubit^{®} 2.0 fluorimeter with Qubit^{®} microRNA Assay kit (Invitrogen, Cat.No. Q32880). Small RNA transcripts were converted to barcoded cDNA libraries. Library preparation was performed as previously reported (Pardini et al., 2018) using the NEBNext Multiplex small RNA Library Prep Set (Cat. No. NEB#E7560) for Illumina and run on NextSeq500 (Illumina Inc., USA). The output of the NextSeq500 Illumina sequencer was demultiplexed using the bcl2fastq Illumina software integrated into the docker4seq package (Cordero et al., 2012; Pardini et al., 2018). Quantification of miRNA expression was performed using the previously described workflow (Pardini et al., 2018) and implementation as described (Beccuti et al., 2018). Sequences were mapped using SHRIMP (Rumble et al., 2009) to Canis familiaris precursor miRNAs available in miRBase 22.1 (http://www.mirbase.org/). Counts tables and CPM (counts per million reads) tables were used to perform differential expression (DE) analysis using as a threshold an adjusted P-value ≤ 0.1 and an absolute log2 Fold Change (log2FC) ≥ 1.

### Computational and statistical analysis

The quality control of raw readings, clipping of adapters, and mapping were performed as previously reported (Cordero et al., 2012). After the readings were mapped, a matrix of integer values was created. The value in the i-th row and j-th column of the matrix shows how many reads were unambiguously assigned to the mature miRNA i in sample j. The undesirable variation present in the data was estimated using the functions implemented in the SVA package (Leek et al., 2012). Differential expression analysis was performed using DESeq2, setting an adjusted p-value threshold < 0.1 and -1 ≥ | log2FC| ≥ 1. Statistical analysis was performed using SPSS 28 (SPSS Inc., Chicago, IL, USA).

### Results

### Demographic Features

The mean age of the subjects enrolled in the study was as follows: ACVIM class A 3.1 years (min. 0.95 - max. 11.3 years); ACVIM class B1 4.2 years (min. 1.1 - max. 9.6 years); ACVIM class B2 8.2 years (min. 3.9 - max. 10 years). The mean body weight was: ACVIM class A 8.3 kg (min. 6.4 - max. 12.4 kg); ACVIM class B1 9 kg (min. 5.8 - max. 12.5 kg); ACVIM class B2 9.3 kg (min. 5.8 - max. 12.1 kg). The whole females were 15, the sterilized females 14, the whole males 17 and castrated 2. The data for each patient are reported in Table 1.

**Table 1**

| **Paziente** | **STADIO** | **ETÀ ANNI** | **PESO** | **SOFFIO** | **SESSO** | **Paziente** | **STADIO** | **ETÀ ANNI** | **PESO** | **SOFFIO** | **SESSO** |
|---|---|---|---|---|---|---|---|---|---|---|---|
| **1** | A | 4,27 | 6,8 | 0 | FS | **25** | B1 | 2,28 | 8,3 | 0 | M |
| **2** | A | 2,05 | 8 | 0 | FS | **26** | B1 | 1,88 | 9,9 | 1 | M |
| **3** | A | 2,32 | 10 | 0 | F | **27** | B1 | 1,77 | 8,2 | 0 | M |
| **4** | A | 5,58 | 12,4 | 0 | MC | **28** | B1 | 2,04 | 8,3 | 1 | F |
| **5** | A | 1,04 | 6,9 | 0 | F | **29** | B1 | 4,57 | 7,1 | 0 | M |
| **6** | A | 0,95 | 7,4 | 0 | FS | **30** | B1 | 8,35 | 9 | 1 | FS |
| **7** | A | 1,44 | 7,9 | 0 | M | **31** | B1 | 9,62 | 9,9 | 1 | FS |
| **8** | A | 11,33 | 7 | 0 | FS | **32** | B1 | 1,36 | 5,8 | 1 | F |
| **9** | A | 1,28 | 9,8 | 0 | M | **33** | B1 | 2,05 | 10 | 0 | M |
| **10** | A | 3,68 | 6,4 | 0 | FS | **34** | B1 | 5,04 | 10,5 | 1 | F |
| **11** | A | 2,31 | 8,6 | 0 | FS | **35** | B1 | 8,02 | 9,5 | 1 | M |
| **12** | A | 1,23 | 8,6 | 0 | F | **36** | B1 | 2,84 | 9 | 1 | M |
| **13** | B1 | 1,16 | 8,6 | 0 | FS | **37** | B2 | 9,31 | 12,1 | 2 | FS |
| **14** | B1 | 4,74 | 10,7 | 0 | M | **38** | B2 | 3,91 | 8,9 | 3 | M |
| **15** | B1 | 2,25 | 8,3 | 1 | F | **39** | B2 | 8,66 | 7,3 | 2 | FS |
| **16** | B1 | 7,23 | 9,3 | 0 | F | **40** | B2 | 7,99 | 10,8 | 1 | F |
| **17** | B1 | 9,60 | 8,6 | 1 | F | **41** | B2 | 7,23 | 8,1 | 2 | M |
| **18** | B1 | 1,71 | 8,9 | 1 | M | **42** | B2 | 9,84 | 8,6 | 2 | F |
| **19** | B1 | 2,33 | 12,5 | 1 | M | **43** | B2 | 9,97 | 7,1 | 2 | F |
| **20** | B1 | 5,23 | 9 | 1 | FS | **44** | B2 | 8,42 | 10,6 | 3 | FS |
| **21** | B1 | 4,73 | 10,6 | 1 | M | **45** | B2 | 7,95 | 10,2 | 2 | F |
| **22** | B1 | 6,30 | 9,8 | 1 | M | **46** | B2 | 8,74 | 10,6 | 2 | F |
| **23** | B1 | 5,40 | 7,7 | 0 | M | **47** | B2 | 9,68 | 5,8 | 2 | FS |
| **24** | B1 | 1,54 | 7,4 | 0 | F | **48** | B2 | 6,38 | 12 | 2 | MC |

Table 1. Data relating to the patients enrolled in the study and classified according to ACVIM. M=male; MC= castrated male; F= female; FS= sterilized female.

Characterization of the circulating miRName and identification of the differentially expressed miRNAs.

In order to characterize the profile of circulating miRNAs in the plasma of dogs from the ACVIM group A (12 samples), B1 (24 samples) and B2 (12 samples), sequencing was performed on the 48 samples. The number of reads for each sample ranged from 361 to 472 and 56 to 458 to 336. Samples 3, 24 and 26 were excluded from further analysis, as the number of reads mapped was too low, respectively 6017, 4009 and 3876. The counts of reads and mapped reads related to each sample are shown in Table 2.

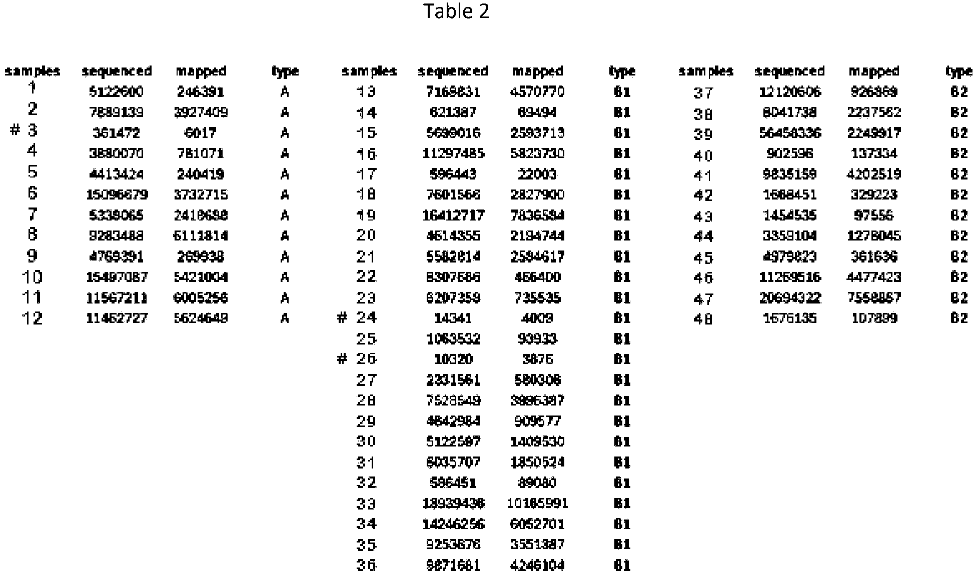

Table 2. Number of reads and reads mapped relative to each sample. Samples indicated with # have been excluded from subsequent analyses due to the low number of reads mapped.

An analysis was carried out to identify the differentially expressed miRNAs (DE-miRNAs) comparing the three ACVIM groups; the analysis identified: 15 DE-miRNAs between B1 and A (P value < 0.05; - 1| Log2FoldChange |+1); 40 DE-miRNAs between B2 and A (P value < 0.05; -1| Log2FoldChange |+1); 46 DE-miRNAs between B2 and B1 (P value < 0.05; -1| Log2FoldChange |+1). Further statistical analysis was then carried out using anovaLike and Deseq2. The DE-miRNAs were a total of 16, as shown in Table 3 which shows the differentially expressed miRNAs and their sequences.

**Table 3**

| **SEQ. ID. NO.** | **NAME** | **SEQUENCE** | **ST 26 STANDARD** |
|---|---|---|---|
| **SEQ. ID NO. 1** | cfa-miR-138b-5p | AGCUGGUGUUGUGAAUCAUGCCGA | AGCTGGTGTTGTGAATCATGCCGA |
| **SEQ. ID NO.2** | cfa-miR-144-5p | GGAUAUCAUCAUAUACUGUAAG | GGATATCATCATATACTGTAAG |
| **SEQ. ID NO.3** | cfa-miR-150-5p | UCUCCCAACCCUUGUACCAGUG | TCTCCCAACCCTTGTACCAGTG |
| **SEQ. ID NO.4** | cfa-miR-182-3p | UGGUUCUAGACUUGCCAACUA | TGGTTCTAGACTTGCCAACTA |
| **SEQ. ID NO.5** | cfa-miR-206-3p | UGGAAUGUAAGGAAGUGUGUGGG | TGGAATGTAAGGAAGTGTGTGG |
| **SEQ. ID NO.6** | cfa-miR-207-3p | GCUUCUCCGGUCUCUCCUCCUUC | GCTTCTCCGGTCTCTCCTCCTTC |
| **SEQ. ID NO.7** | cfa-miR-33b-5p | GUGCAUUGCUGUUGCAUUGC | GTGCATTGCTGTTGCATTGC |
| **SEQ. ID NO.8** | cfa-miR-346-5p | UGUCUGCCCGCAUGCCUGCCUCU | TGTCTGCCCGCATGCCTGCCTCT |
| **SEQ. ID NO.9** | cfa-miR-365-1-5p | AGGGACUUUUGGGGGCAGAU | AGGGACTTTTGGGGGCAGAT |
| **SEQ. ID NO.10** | cfa-miR-493-5p | UGUACAUGGUAGGCUUUCAUU | TGTACATGGTAGGCTTTCATT |
| **SEQ. ID NO.11** | cfa-miR-632-5p | CUGACGGGAGCAGAGCGGCGAA | CTGACGGGAGCAGAGCGGCGAA |
| **SEQ. ID NO.12** | cfa-miR-8794-1-5p | AAUCGGCAAGUGGCCUGGGGAC | AATCGGCAAGTGGCCTGGGGAC |
| **SEQ. ID NO.13** | cfa-miR-8875-3p | AUUGUCUUUGGGUUCUUAGCCU | ATTGTCTTTGGGTTCTTAGCCT |
| **SEQ. ID NO.14** | cfa-miR-92b-3p | UAUUGCACUCGUCCCGGCCUCC | TATTGCACTCGTCCCGGCCTCC |
| **SEQ. ID NO.15** | cfa-miR-92b-5p | AGGGACGGGACGCGGUGCAGU | AGGGACGGGACGCGGTGCAGT |
| **SEQ. ID NO.16** | cfa-miR-95-3p | UUCAACGGGUAUUUAUUGAGCA | TTCAACGGGTATTTATTGAGCA |

Differentially expressed miRNAs in the various groups were also identified as shown in Table 4 which sets forth the list of the differentially expressed miRNAs in the various ACVIM and Log2FoldChange (Log2FC) groups relative to their expression.

Log2FC represents the logarithm (base 2) of the ratio of expression levels between two conditions (in this case sample versus standard/control).

It can be calculated with the following formula: $$ *\text{Log2 Fold Change} = \log_2 \left( \frac{\text{Expression Level in Condition A}}{\text{Expression Level in Condition B}} \right) $$*

Positive values indicate up-regulation, negative values indicate down-regulation while the zero value means that there is no difference in expression levels between the two conditions.

For example, conventionally Log2FC = 1 means doubled expression (hence over-expression), Log2FC = -1: means halved expression (under-expression) and Log2FC = 0 means no change in expression.

The results are shown in Table 4:
- in the ACVIM B1 group the expression of cfa-miR-493-5p SEQ.ID NO. 10 increases (Log₂FC= 1.77) compared to the ACVIM A group, while the expression of the cfa-miR-144-5p miRNAs SEQ.ID NO. 2 (Log₂HR= -1.95) , cfa-miR-206-3p SEQ.ID NO.5 (Log₂HR= -2.35) , cfa-miR-207-3p SEQ.ID NO.6 (Log₂HR= -1.38) , cfa-miR-365-1-5p SEQ.ID NO.9 (Log₂HR= -1.79), cfa-miR-632-5p SEQ.ID NO.11 (Log₂HR= -3.23) decreases;
- in young ACVIM B1 subjects (age<3 years) three miRNAs, cfa-miR-207-3p SEQ.ID NO.6 (Log₂FC= -1.22), cfa-miR-33b-5p SEQ.ID NO.7 (Log₂FC= -1.78) and cfa-miR-632-5p SEQ.ID NO.11 (Log₂FC= -1.68) decrease compared to ACVIM A;
- in the ACVIM B2 group the expression of cfa-miR-182-3p SEQ.ID NO.4 (Log₂FC= 1.82) , cfa-miR-493-5p SEQ.ID NO.10 (Log₂FC= 3.42), cfa-miR-8794-1-5p SEQ.ID NO.12 (Log₂FC= 12.33) , cfa-miR-8875-3p SEQ.ID NO.13 (Log₂FC= 2.41) increases, while that of cfa-miR-144-5p SEQ.ID NO.2 (Log₂FC= -3.2), cfa-miR-150-5p SEQ.ID NO.3(Log₂FC= -1.36), cfa-miR-206-3p SEQ.ID NO.5 (Log₂FC= -2.51), cfa-miR-365-1-5p SEQ.ID NO.9 (Log₂FC= -1.14), cfa-miR-632-5p SEQ.ID NO.11 (Log₂FC= -4.02), decreases compared to the ACVIM A group;
- in the ACVIM B2 group the expression of cfa-miR-150-5p SEQ.ID NO.3 (Log₂FC= -1.23) dims, while that of cfa-miR-138b-5p SEQ.ID NO.1 (Log₂FC= 1.72), cfa-miR-346-5p SEQ.ID NO.8 (Log₂FC= 1.86), cfa-miR-8875-3p SEQ.ID NO. 13 (Log₂FC= 1.82), cfa-miR-92b-3p SEQ.ID NO. 14 (Log₂FC= 1.35), cfa-miR-92b-5p SEQ.ID NO. 15 (Log₂HR= 1.1), cfa-miR-95-3p SEQ.ID NO. 16 (Log₂HR= 1.49) increases compared to ACVIM B1 group.

**Table 4**

| | **miRNA** | **B1 vs A (LogzFC)** | **B2 vs A (Log₂FC)** | **B1 < 3 years vs A (Log₂FC)** | **B2 vs B1 (LogzFC)** |
|---|---|---|---|---|---|
| **SEQ. ID NO. 1** | cfa-miR-138b-5p | | | | 1.72 |
| **SEQ. ID NO.2** | cfa-miR-144-5p | -1.95 | -3.2 | | |
| **SEQ. ID NO.3** | cfa-miR-150-5p | | -1.36 | | -1.2 |
| **SEQ. ID NO.4** | cfa-miR-182-3p | | 1.82 | | |
| **SEQ. ID NO.5** | cfa-miR-206-3p | -2.35 | -2.51 | | |
| **SEQ. ID NO.6** | cfa-miR-207-3p | -1.38 | | -1.22 | |
| **SEQ. ID NO.7** | cfa-miR-33b-5p | | | -1.78 | |
| **SEQ. ID NO.8** | cfa-miR-346-5p | | | | 1.86 |
| **SEQ. ID NO.9** | cfa-miR-365-1-5p | -1.79 | -1.14 | | |
| **SEQ. ID NO.10** | cfa-miR-493-5p | 1.77 | 3.42 | | |
| **SEQ. ID NO.11** | cfa-miR-632-5p | -3.23 | -4.02 | -1.68 | |
| **SEQ. ID NO.12** | cfa-miR-8794-1-5p | | 2.33 | | |
| **SEQ. ID NO.13** | cfa-miR-8875-3p | | 2.41 | | 1.82 |
| **SEQ. ID NO.14** | cfa-miR-92b-3p | | | | 1.35 |
| **SEQ. ID NO.15** | cfa-miR-92b-5p | | | | 1.1 |
| **SEQ. ID NO.16** | cfa-miR-95-3p | | | | 1.4 |

### Bibliography

Acierno, M. J., Brown, S., Coleman, A. E., Jepson, R. E., Papich, M., Stepien, R. L., & Syme, H. M., 2018, ACVIM consensus statement: Guidelines for the identification, evaluation, and management of systemic hypertension in dogs and cats. Journal of Veterinary Internal Medicine, 32(6), 1803-1822. https://doi.org/10.1111/jvim.15331
Bagardi M, Ghilardi S, Zamarian V, Ceciliani F, Brambilla PG, Lecchi C, 2002 Circulating MiR-30b-5p is upregulated in Cavalier King Charles Spaniels affected by early myxomatous mitral valve disease. PLoS One, Jul 11;17(7):e0266208. doi: 10.1371/journal.pone.0266208.
Beccuti, M., Cordero, F., Arigoni, M., Panero, R., Amparore, E. G., Donatelli, S., & Calogero, R. A, 2018, SeqBox: RNAseq/ChlPseq reproducible analysis on a consumer game computer. Bioinformatics, 34(5), 871-872. https://doi.org/10.1093/bioinformatics/btx674
Bustin, S. A., Benes, V., Garson, J. A., Hellemans, J., Huggett, J., Kubista, M., Mueller, R., Nolan, T., Pfaffl, M. W., Shipley, G. L., Vandesompele, J., & Wittwer, C. T., 2009, The MIQE Guidelines: Minimum Information for Publication of Quantitative Real-Time PCR Experiments. Clinical Chemistry, 55(4), 611-622. https://doi.org/10.1373/clinchem.2008.112797
Chetboul, V., & Tissier, R. 2012,. Echocardiographic assessment of canine degenerative mitral valve disease. Journal of Veterinary Cardiology : The Official Journal of the European Society of Veterinary Cardiology, 14(1), 127-148. https://doi.org/10.1016/j.jvc.2011.11.005
Cordero, F., Beccuti, M., Arigoni, M., Donatelli, S., & Calogero, R. A. 2012, Optimizing a Massive Parallel Sequencing workflow for quantitative miRNA expression analysis. PLoS ONE, 7(2). https://doi.org/10.1371/journal.pone.0031630
Ghilardi S, Lecchi C,Bagardi M, Romito G, Colombo FM, Polli M, Franco C, Brambilla PG. PLoS One. 2022,Prospective pilot study on the predictive significance of plasma miR-30b-5p through the study of echocardiographic modifications in Cavalier King Charles Spaniels affected by different stages of myxomatous mitral valve disease: The PRIME study. Dec 27;17(12):e0274724. doi: 10.1371/journal.pone.0274724. eCollection 2022. PMID:36574372
Jung S,Bohan A, 2018,Genome-wide sequencing and quantification of circulating microRNAs for dogs with congestive heart failure secondary to myxomatous mitral valve degeneration. Am J Vet Res. Feb;79(2):163-169. doi: 10.2460/ajvr.79.2.163. PMID:29359980
Keene BW, Atkins CE, . Bonagura JD, Fox PR, Häggström J, Fuentes VL, Oyama MA., Rush JE, Stepien R, Uechi M, ACVIM consensus guidelines for the diagnosis and treatment of myxomatous mitral valve disease in dogs, 2019, J Vet Intern Med, 33:1127-1140.
Leek, J. T., Johnson, W. E., Parker, H. S., Jaffe, A. E., & Storey, J. D. 2012, The sva package for removing batch effects and other unwanted variation in high-throughput experiments. Bioinformatics (Oxford, England), 28(6), 882-883. https://doi.org/10.1093/bioinformatics/bts034
Li Q, Freeman LM, Rush JE, Laflamme DP, 2015, Expression Profiling of Circulating MicroRNAs in Canine Myxomatous Mitral Valve Disease. Int J Mol Sci. Jun 19;16(6):14098-108. doi:10.3390/ijms160614098. PMID: 26101868
Pardini, B., Cordero, F., Naccarati, A., Viberti, C., Birolo, G., Oderda, M., Di Gaetano, C., Arigoni, M., Martina, F., Calogero, R. A., Sacerdote, C., Gontero, P., Vineis, P., & Matullo, G.,2018, microRNA profiles in urine by next-generation sequencing can stratify bladder cancer subtypes. Oncotarget, 9(29). https://doi.org/10.18632/oncotarget.25057
Ponikowski, P., Voors, A. A., Anker, S. D., Bueno, H., Cleland, J. G. F., Coats, A. J. S., Falk, V., González-Juanatey, J. R., Harjola, V.-P., Jankowska, E. A., Jessup, M., Linde, C., Nihoyannopoulos, P., Parissis, J. T., Pieske, B., Riley, J. P., Rosano, G. M. C., Ruilope, L. M., Ruschitzka, F., ... ESC Scientific Document Group. 2016, 2016 ESC Guidelines for the diagnosis and treatment of acute and chronic heart failure: The Task Force for the diagnosis and treatment of acute and chronic heart failure of the European Society of Cardiology (ESC)Developed with the special contribution of the Heart Failure Association (HFA) of the ESC. European Heart Journal, 37(27), 2129-2200. https://doi.org/10.1093/eurheartj/ehw128
Rishniw, M. 2018,. Murmur grading in humans and animals: past and present. Journal of Veterinary Cardiology, 20(4), 223-233. https://doi.org/10.1016/j.jvc.2018.06.001
Ro WB, Kang MH, Song DW, Kim HS, Lee GW, Park HM, 2021, Identification and Characterization of Circulating MicroRNAs as Novel Biomarkers in Dogs With Heart Diseases. Front Vet Sci. Oct 11;8:729929. doi: 10.3389/fvets.2021.729929. eCollection 2021. PMID: 34708100
Rumble, S. M., Lacroute, P., Dalca, A. V., Fiume, M., Sidow, A., & Brudno, M. 2009, SHRiMP: Accurate Mapping of Short Color-space Reads. PLoS Computational Biology, 5(5), e1000386. https://doi.org/10.1371/journal.pcbi.1000386
Thomas W. P., Gaber, C. E., Jacobs, G. J., Kaplan, P. M., Lombard, C. W., Moise, N. S., & Moses, B. L. 1993. Recommendations for standards in transthoracic two-dimensional echocardiography in the dog and cat. Echocardiography Committee of the Specialty of Cardiology, American College of Veterinary Internal Medicine. Journal of Veterinary Internal Medicine, 7(4), 247-252. https://doi.org/10.1111/j.1939-1676.1993.tb01015.x
Yang VK, et al. 2017, Circulating exosome microRNA associated with heart failure secondary to myxomatous mitral valve disease in a naturally occurring canine model. J Extracell Vesicles.. PMID: 28804599
Yang VK, Tai AK, Huh TP, Meola DM,Juhr CM, Robinson NA, Hoffman AM, 2018, Dysregulation of valvular interstitial cell let-7c, miR-17, miR-20a, and miR-30d in naturallyoccurring canine myxomatous mitral valve disease. PLoS One. Jan 9;13(1):e0188617. doi:10.1371/journal.pone.0188617. eCollection 2018. PMID: 29315310
Palarea-Albaladejo Javier et al., "Assessing the use of blood microRNA expression patterns for predictive diagnosis of myxomatous mitral valve disease in dogs", Frontiers In Veterinary Science, vol. 11, 1 November 2024 (2024-11-01), page 1443847, Lausanne, DOI: 10.3389/fvets.2024.1443847

## Claims

1. A miRNA panel consisting of the following miRNAs:
| **SEQ. ID. NO.** | **NAME** | **SEQUENCE** | **ST 26 STANDARD** |
|---|---|---|---|
| **SEQ. ID NO. 1** | cfa-miR-138b-5p | AGCUGGUGUUGUGAAUCAUGCCGA | AGCTGGTGTTGTGAATCATGCCG A |
| **SEQ. ID NO.2** | cfa-miR-144-5p | GGAUAUCAUCAUAUACUGUAAG | GGATATCATCATATACTGTAAG |
| **SEQ. ID NO.3** | cfa-miR-150-5p | UCUCCCAACCCUUGUACCAGUG | TCTCCCAACCCTTGTACCAGTG |
| **SEQ. ID NO.4** | cfa-miR-182-3p | UGGUUCUAGACUUGCCAACUA | TGGTTCTAGACTTGCCAACTA |
| **SEQ. ID NO.5** | cfa-miR-206-3p | UGGAAUGUAAGGAAGUGUGUGGG | TGGAATGTAAGGAAGTGTGTGG |
| **SEQ. ID NO.6** | cfa-miR-207-3p | GCUUCUCCGGUCUCUCCUCCUUC | GCTTCTCCGGTCTCTCCTCCTTC |
| **SEQ. ID NO.7** | cfa-miR-33b-5p | GUGCAUUGCUGUUGCAUUGC | GTGCATTGCTGTTGCATTGC |
| **SEQ. ID NO.8** | cfa-miR-346-5p | UGUCUGCCCGCAUGCCUGCCUCU | TGTCTGCCCGCATGCCTGCCTCT |
| **SEQ. ID NO.9** | cfa-miR-365-1-5p | AGGGACUUUUGGGGGCAGAU | AGGGACTTTTGGGGGCAGAT |
| **SEQ. ID NO.10** | cfa-miR-493-5p | UGUACAUGGUAGGCUUUCAUU | TGTACATGGTAGGCTTTCATT |
| **SEQ. ID NO.11** | cfa-miR-632-5p | CUGACGGGAGCAGAGCGGCGAA | CTGACGGGAGCAGAGCGGCGAA |
| **SEQ. ID NO.12** | cfa-miR-8794-1-5p | AAUCGGCAAGUGGCCUGGGGAC | AATCGGCAAGTGGCCTGGGGAC |
| **SEQ. ID NO.13** | cfa-miR-8875-3p | AUUGUCUUUGGGUUCUUAGCCU | ATTGTCTTTGGGTTCTTAGCCT |
| **SEQ. ID NO.14** | cfa-miR-92b-3p | UAUUGCACUCGUCCCGGCCUCC | TATTGCACTCGTCCCGGCCTCC |
| **SEQ. ID NO.15** | cfa-miR-92b-5p | AGGGACGGGACGCGGUGCAGU | AGGGACGGGACGCGGTGCAGT |
| **SEQ. ID NO.16** | cfa-miR-95-3p | UUCAACGGGUAUUUAUUGAGCA | TTCAACGGGTATTTATTGAGCA |

2. A miRNA panel according to claim 1 selected from the group consisting of:
cfa-miR-144-5p SEQ. ID NO.2, cfa-miR-206-3p SEQ. ID NO.5, cfa-miR-207-3p SEQ. ID NO.6, cfa-miR-365-1-5p SEQ. ID NO.9, cfa-miR-493-5p SEQ. ID NO.10, cfa-miR-632-5p SEQ. ID NO.11;
cfa-miR-207-3p SEQ. ID NO.6, cfa-miR-33b-5p SEQ. ID NO.7, and cfa-miR-632-5p SEQ. ID NO.11;
cfa-miR-144-5p SEQ. ID NO.2, cfa-miR-150-5p SEQ. ID NO.3, cfa-miR-182-3p SEQ. ID NO.4, cfa-miR-206-3p SEQ. ID NO.5, cfa-miR-365-1-5p SEQ. ID NO.9, cfa-miR-493-5p SEQ. ID NO.10, cfa-miR-632-5p SEQ. ID NO.11, cfa-miR-8794-1-5p SEQ. ID NO.12, cfa-miR-8875-3p SEQ. ID NO.13;
cfa-miR-138b-5p SEQ. ID NO.1, cfa-miR-150-5p SEQ. ID NO.3, cfa-miR-346-5p SEQ. ID NO.8, cfa-miR-8875-3p SEQ. ID NO.13, cfa-miR-92b-3p SEQ. ID NO.14, cfa-miR-92b-5p SEQ. ID NO.15, cfa-miR-95-3p SEQ. ID NO.16.

3. Use of at least one miRNA of claim 1 preferably at least three miRNAs, more preferably all as a diagnostic marker, preferably of myxomatous mitral valve disease, preferably in dogs.

4. The use according to claim 3 wherein at least one miRNA, preferably at least three miRNAs, more preferably all miRNAs from the group consisting of: from SEQ.ID.NO.1 to SEQ.ID.NO. 16 as a diagnostic marker of absence of MMVD or healthy subject or subject MMVD A.

5. The use according to claim 3 wherein at least one miRNA, preferably at least three miRNAs, more preferably all miRNAs from the group consisting of: cfa-miR-144-5p SEQ.ID.NO.2, cfa-miR-206-3p SEQ.ID.NO.5, cfa-miR-207-3p SEQ.ID.NO.6, cfa-miR-365-1-5p SEQ.ID.NO.9, cfa-miR-493-5p SEQ.ID.NO.10, cfa-miR-632-5p SEQ.ID.NO.11 as a diagnostic marker of MMVD B1.

6. The use according to claim 3 wherein at least one miRNA, preferably at least three miRNAs, more preferably all miRNAs from the group consisting of: cfa-miR-207-3p SEQ.ID.NO.6, cfa-miR-33b-5p SEQ.ID.NO.7, and cfa-miR-632-5p SEQ.ID.NO.11, is a diagnostic marker of MMVD B1 in subjects under 3 years of age.

7. The use according to claim 3 wherein at least one miRNA, preferably at least three miRNAs, more preferably all miRNAs from the group consisting of: cfa-miR-144-5p SEQ.ID.NO.2, cfa-miR-150-5p SEQ.ID.NO.3, cfa-miR-182-3p SEQ.ID.NO.4, cfa-miR-206-3p SEQ.ID.NO.5, cfa-miR-365-1-5p SEQ.ID.NO.9, cfa-miR-493-5p SEQ.ID.NO.10, cfa-miR-632-5p SEQ.ID.NO.11, cfa-miR-8794-1-5p SEQ.ID.NO.12, cfa-miR-8875-3p SEQ.ID.NO.13 is diagnostic marker of MMVD B2.

8. Use according to claim 3 wherein at least one miRNA, preferably at least three miRNAs, more preferably all miRNAs from the group consisting of: cfa-miR-138b-5p SEQ.ID.NO.1, cfa-miR-150-5p SEQ.ID.NO.3, cfa-miR-346-5p SEQ.ID.NO.8, cfa-miR-8875-3p SEQ.ID.NO.13, cfa-miR-92b-3p SEQ.ID.NO.14, cfa-miR-92b-5p SEQ.ID.NO.15, cfa-miR-95-3p SEQ.ID.NO.16, is discriminant diagnostic marker of MMVD B1 versus MMVD B2.

9. A method of diagnosing the risk of suffering, the presence or absence and the degree of development or severity of mitral valve myxomatous disease which involves identifying and/or measuring the expression level of at least one miRNA, preferably three miRNAs, selected from the group consisting of: SEQ.ID.NO.1 to SEQ.ID.NO.16 in an isolated sample of biological fluid obtained from said subject wherein the presence and/or the modified level of expression of said at least one miRNA relative to a control, indicates an increased risk to the subject of suffering, the presence or absence, the degree of development or severity of mitral valve myxomatous disease, wherein said subject is a dog and wherein the control is an isolated sample obtained from a healthy subject or MMVD A or MMVD B1

10. The method according to claim 9 comprising the following steps:
a) collection of an isolated sample of biological fluid from a subject at risk or with MMVD
b) extract the miRNAs from the sample obtained in stage a)
c) amplification of the individual miRNAs extracted in stage b), preferably by quantitative PCR
d) measurement of the level of expression of at least one miRNA selected from the group consisting of: from SEQ.ID.NO.1 to SEQ.ID.NO. 16
e) compare the presence and/or the modified expression level of said at least one miRNA referred to in step d) with respect to a control
wherein the presence and/or modified level of expression of at least one miRNA of step d) relative to the control indicates an increased risk to the subject of suffering from, the presence or absence, degree of development or severity of myxomatous disease of the mitral valve, wherein the control is an isolated sample obtained from a healthy subject or MMVD A or MMVD B1.

11. The method of anyone of claims 9 and 10, wherein the variation in the expression level of at least one miRNA, preferably all miRNAs, selected from the group consisting of: cfa-miR-144-5p SEQ.ID.NO.2, cfa-miR-206-3p SEQ.ID.NO.5, cfa-miR-207-3p SEQ.ID.NO.6, cfa-miR-365-1-5p SEQ.ID.NO.9, cfa-miR-493-5p SEQ.ID.NO.10, cfa-miR-632-5p SEQ.ID.NO.11, is indicative of MMVD B1.

12. The method of anyone of claims 9 and 10 wherein the variation in the expression level of at least one miRNA, preferably all miRNAs, selected from the group consisting of: cfa-miR-207-3p SEQ.ID.NO.6, cfa-miR-33b-5p SEQ.ID.NO.7, and cfa-miR-632-5p SEQ.ID.NO.11, is indicative of MMVD B1 in subjects under 3 years of age.

13. The method of anyone of claims 9 and 10 wherein the variation in the expression level of at least one miRNA, preferably all miRNAs, selected from the group consisting of: cfa-miR-144-5p SEQ.ID.NO.2, cfa-miR-150-5p SEQ.ID.NO.3, cfa-miR-182-3p SEQ.ID.NO.4, cfa-miR-206-3p SEQ.ID.NO.5, cfa-miR-365-1-5p SEQ.ID.NO.9, cfa-miR-493-5p SEQ.ID.NO.10, cfa-miR-632-5p SEQ.ID.NO.11, cfa-miR-8794-1-5p SEQ.ID.NO.12, cfa-miR-8875-3p SEQ.ID.NO.13 is indicative of MMVD B2.

14. The method according to anyone of claims 9 and 10, wherein the variation in the expression level of all miRNAs cfa-miR-138b-5p SEQ.ID.NO.1, cfa-miR-150-5p SEQ.ID.NO.3, cfa-miR-346-5p SEQ.ID.NO.8, cfa-miR-8875-3p SEQ.ID.NO.13, cfa-miR-92b-3p SEQ.ID.NO.14, cfa-miR-92b-5p SEQ.ID.NO.15, cfa-miR-95-3p SEQ.ID.NO.16, allows to discriminate MMVD to B1 subjects from MMVD B2 subjects.

15. A method according to claim 11 wherein cfa-miR-493-5p SEQ.ID NO. 10 is overexpressed and cfa-miR-144-5p SEQ.ID NO. 2, cfa-miR-206-3p SEQ.ID NO.5, cfa-miR-207-3p SEQ.ID NO.6, cfa-miR-365-1-5p and cfa-miR-632-5p SEQ.ID NO.11 are under-expressed relative to the control.

16. Method according to claim 12 wherein cfa-miR-207-3p SEQ.ID.NO.6, cfa-miR-33b-5p SEQ.ID.NO.7, cfa-miR-632-5p SEQ.ID.NO.11 are under-expressed compared to the control.

17. The method according to claim 13 wherein cfa-miR-182-3p SEQ.ID NO.4, cfa-miR-493-5p SEQ.ID NO.10, cfa-miR-8794-1-5p SEQ.ID NO.12, cfa-miR-8875-3p SEQ.ID NO.13 are overexpressed and cfa-miR-144-5p SEQ.ID NO.2), cfa-miR-150-5p SEQ.ID NO.3, cfa-miR-206-3p SEQ.ID NO.5, cfa-miR-365-1-5p SEQ.ID NO.9, cfa-miR-632-5p SEQ.ID NO.11 are under-expressed relative to the control.

18. The method according to claim 14, wherein cfa-miR-150-5p SEQ.ID NO.3 is under expressed and cfa-miR-138b-5p SEQ.ID NO.1, cfa-miR-346-5p SEQ.ID NO.8, cfa-miR-8875-3p SEQ.ID No. 13, cfa-miR-92b-3p SEQ.ID NO. 14, cfa-miR-92b-5p SEQ.ID NO. 15, cfa-miR-95-3p SEQ.ID NO. 16 are overexpressed compared to MMVD B1 stage.

19. Kit for the in vitro evaluation of the risk of suffering from, the presence or absence and the degree of development or severity of mitral valve myxomatous disease comprising swabs and reagents for extracting, amplifying and measuring the expression level of at least one miRNA selected from the group consisting of: SEQ.ID NO.1 TO SEQ.ID NO.16 and comparing it to a control by the method of any one of claims 9 to 18.
